# EUROPEAN PATENT SPECIFICATION

(11) **EP 3 287 768 B1**
(45) Date of publication and mention of the grant of the patent: **09.08.2023**
(21) Application number: 17188082.6
(22) Date of filing: 28.08.2017
(51) Int. Cl.: G01N 21/552, C12Q 1/68, B82Y 15/00, G01N 21/85, G01N 21/77, C12Q 1/6825, G01N 33/543

(54) **DETECTION OF DISTINCT MOLECULES USING FIBER OPTIC SENSORS**
ERKENNUNG VON UNTERSCHIEDLICHEN MOLEKÜLEN MIT GLASFASERSENSOREN
DÉTECTION DE MOLÉCULES DISTINCTES À L'AIDE DE CAPTEURS À FIBRE OPTIQUE

(30) Priority: 26.08.2016 GB 201614597
(43) Date of publication of application: 28.02.2018
(73) Proprietor: FOx Biosystems NV, 3590 Diepenbeek (BE)
(72) Inventor: LAMMERTYN, Jeroen, 3040 Neerijse (BE); DELPORT, Filip, 9140 Temse (BE); DAEMS, Devin, 2850 Boom (BE)
(74) Representative: Arnold & Siedsma

(56) References cited:
- WO-A1-2015/071338
- US-A1- 2006 286 680
- JIADI LU ET AL: "Fiber optic-SPR platform for fast and sensitive infliximab detection in serum of inflammatory bowel disease patients", BIOSENSORS AND BIOELECTRONICS, vol. 79, May 2016 (2016-05), pages 173-179, XP055431810, NL ISSN: 0956-5663, DOI: 10.1016/j.bios.2015.11.087
- BENIAMINO SCIACCA ET AL: "Dip Biosensor Based on Localized Surface Plasmon Resonance at the Tip of an Optical Fiber", LANGMUIR, vol. 30, no. 3, 15 January 2014 (2014-01-15), pages 946-954, XP055432381, US ISSN: 0743-7463, DOI: 10.1021/la403667q
- Karel Knez ET AL: "Spherical Nucleic Acid Enhanced FO-SPR DNA Melting for Detection of Mutations in Legionella pneumophila", Analytical Chemistry, vol. 85, no. 3, 5 February 2013 (2013-02-05), pages 1734-1742, XP055580936, US ISSN: 0003-2700, DOI: 10.1021/ac303008f

## Description

### Field of the invention

The invention relates to the field of detection of molecules in a sample using a mass-sensitive sensor. More specifically it relates to a method for detecting a plurality of distinct target molecules in a sample fluid using at least one fiber optic sensor, a fiber optic sensor adapted for detecting a plurality of distinct molecules in a sample.

### Background of the invention

A fast, simple and reliable analysis of molecules, e.g. biomolecules, such as the detection and/or quantification of various proteins in a sample, can be important in research and clinical applications. Diagnostic tests for allergens, contaminants or diseases may rely on such analysis. For example, a pathological condition may be indicated by the presence of small quantities of specific biomolecules. Multiplex assays are known in the art for detecting and/or quantifying a plurality of different analytes in a sample in a single test operation.

For nucleic acid analysis, techniques such as DNA melting analysis using high resolution instrumentation and specialized fluorescent DNA-binding dyes are used to determine the presence and identity of different nucleic acids in the same solution. Another technique based on qPCR powerfully combines sensitive nucleic acid detection in real-time with multiplexing capacity, e.g. as disclosed by Espy et al., Clin Microbiol Rev, 2006, 1 9, pp. 165-256. However, these techniques require the use of multiple fluorescent dyes to monitor the amplification of target nucleic acids in real-time. Because resonance energy transfer occurs between the dyes, the sensitivity of these reactions is hampered, and most qPCR assays can simultaneously detect only 2 or 3 targets. Conventional PCR performed without dyes could support many more targets, as evidenced by experiments in which the characteristic melting temperature (Tₘ) of PCR amplicons were used as a secondary label in qPCR, leading to identification of 50 different DNA sequences in one sample, e.g. as disclosed by Mackay et al. in Nucleic acids res., 2002, 30, pp. 1292- 1305.

For example, it is known in the art to perform a solid phase PCR reaction using a first primer which is attached to a sensitive surface of the sensor and a second primer which is attached to a gold particle. In such prior art approach, multiplex PCR reactions may be achieved by using multiple sensor probes, e.g. an individually functionalized optic fiber probe for each amplification reaction to be studied.

However, there remains a need in the art for techniques which enable rapid, sensitive, and accurate identification and quantification of molecules, e.g. biomolecules such as proteins.

Furthermore, it is known in the art to monitor DNA melting in real-time using surface plasmon resonance (SPR) devices. For example, an SPR imaging device was used to discriminate between SNPs in short target sequences by Fiche et al., Analytical Chemistry, 2008, 80 , 1049-1057, while hybridization of gold nanoparticles (Au NPs) to the surface of SPR devices was used to improve resolution, so that SNPs were discriminated in longer targets, as disclosed by Knez et al., Small, 2012, 8, 868-872.

It is also known in the art to use a fiber optic SPR (FO-SPR) device as a "dip probe" to test different solutions. An FO-SPR device can be used for monitoring solid phase PCR amplification reactions, e.g. as disclosed by Pollet et al., Small, 2011, 7, 1003-1006. In this method, a solid phase PCR reaction is performed using one primer which is attached to the sensor and one primer which is attached to a gold particle. The probes on the surface may be extended during PCR and determination of the melting point can thus be dictated by the behaviour of the entire amplicon and not just by primer template hybridisation. However, in accordance with this approach, multiplex PCR reactions would be achieved by using multiple fibers, e.g. an individually functionalized fiber for each amplification reaction.

Another fiber optic surface plasmon resonance sensor was disclosed by Pollet et al. in "Fiber optic SPR biosensing of DNA hybridization and DNA-protein interactions," published in Biosens. Bioelectron. 25(4), pp. 864-869. This reusable, label-free fiber optic SPR biosensor can be used for measuring DNA hybridization and DNA-protein interactions. Particularly, a fiber-based SPR system is combined with DNA aptamer bioreceptors.

Furthermore, in "Fiber optic-SPR platform for fast and sensitive infliximab detection in serum of inflammatory bowel disease patients;" Biosens Bioelectron. 79, pp. 173-179, Lu et al. disclosed a fast bioassay for determining infliximab concentration in serum using a fiber-optic surface plasmon resonance (FO-SPR) biosensor, in which an infliximab-specific antibody is covalently immobilized on the sensor surface of the FO-SPR biosensor.

It is furthermore known in the art to detect multiple proteins on a single spot by laser ablation inductively coupled plasma mass spectrometry (LA-ICPMS). For example, Hu et al. disclosed, in "Detection of Multiple Proteins on One Spot by Laser Ablation Inductively Coupled Plasma Mass Spectrometry and Application to Immuno-Microarray with Element-Tagged Antibodies," Anal. Chem. 2007, 79, 923-929, a detection method based on LA-ICPS that allows detecting multiple biological analytes from each spot of a microarray by using sandwich-type immunoreactions on a microarray with element-tagged antibodies, e.g. antibodies conjugated to different metal ions or nanoparticles.

WO2015/071338 A1 discloses detection of a target nucleic acid in a sample using a mass sensitive sensor functionalized with a first nucleic acid probe hybridizing to the target nucleic acid, and metal nanoparticles functionalized with a second nucleic acid probe hybridizing to the target nucleic acid. A nucleic acid amplification of the target nucleic acid is performed using non-immobilized probes complementary to the target nucleic DNA.

### Summary of the invention

It is an object of embodiments of the present invention to provide good and efficient methods and means for detection and/or quantification of different target molecules, e.g. biomolecules, in a sample using a fiber optic sensor, e.g. a fiber optic sensor, e.g. fiber optic biosensor, e.g. for multiplex analyte detection using a fiber optic biosensor.

The above objective is accomplished by a method as defined in appended independent claim 1.

The fiber optic sensor may comprise a configuration configuration as known in the art, e.g. described in Sensors & Bioelectronics Vol. 12. No. 4, pp. 329-336, 1997.

It is an advantage of embodiments of the present invention that target molecules not present in the sample fluid can be eliminated quickly by stepwise testing.

A method according to embodiments of the present invention may further comprise exposing the or any of the at least one fiber optic sensor to a further reagent fluid of the plurality of reagent fluids, and detecting a further signal generated by the thus exposed fiber optic sensor to determine whether at least one further target molecule or any of a further combination of target molecules was present in the sample fluid.

In a method according to embodiments of the present invention, the affinity reagent or affinity reagents in the further reagent fluid may form a second proper subset of the affinity reagents in the first reagent fluid, in which this second proper subset is complimentary to the first proper subset.

In a method according to embodiments of the present invention, the number of affinity reagents in the second proper subset may be in the range of 50% to 150% of the number of affinity reagents in the first proper subset.

It is an advantage of embodiments of the present invention that molecules not present in the sample fluid can be eliminated quickly by stepwise testing for complimentary sets of about equal size.

It is an advantage of embodiments of the present invention that a fiber optic sensor can be easily reused for further testing if no binding was to molecules to be tested was observed.

In a method according to embodiments of the present invention, the at least one fiber optic sensor may comprise a plurality of fiber optic sensors, wherein the second fiber optic sensor is exposed to the second reagent fluid if the first signal indicated a presence of the first target molecule or of any target molecule of the first combination of target molecules.

It is an advantage of embodiments of the present invention that identical fiber optic sensors can be used, while allowing a specific testing for a plurality of molecules of interest in a stepwise procedure. It is a further advantage that distinct fiber optic sensors need not to be manufactured and functionalized specifically to test for different molecules.

In a method according to embodiments of the present invention, the first reagent fluid may comprise, for each target molecule of the plurality of target molecules, a corresponding affinity reagent for binding simultaneously with a corresponding affinity probe of the plurality of distinct affinity probes to the target molecule, such that the first detected signal forms a global screening signal indicative of the presence of any of the plurality of target molecules in the sample liquid.

It is an advantage of embodiments of the present invention that a quick screening can be obtained for a large number of target molecules, while allowing an efficient identification of the present target molecules in a stepwise approach.

In a method according to embodiments of the present invention, the target molecules may comprise proteins to be detected in the sample fluid.

It is an advantage of embodiments of the present invention that an efficient and specific detection of proteins is provided using fiber optic sensor probes, e.g. where disambiguation of binding of different target proteins on the sensor may be difficult using different melting curve characteristics.

In the method according to the present invention, the optical signal is monitored while increasing the temperature of the detection surface (directly or indirectly, for example by heating a fluid, e.g. the sample fluid, in which the detection surface is immersed) and a change in the monitored optical signal, which change is representative of a temperature-induced release of target molecules from the detection surface, is detected to determine whether the target molecules were present in the sample and were bound to the detection surface by a corresponding affinity probe and affinity reagent.

In a method according to embodiments of the present invention, the detection surface of the at least one fiber optic sensor may be provided with a surface texturing structure to enable binding of the target molecules on the corresponding affinity probes in a predetermined orientation.

In a method according to embodiments of the present invention, the surface texturing structure may comprise self-assembled DNA structures, e.g. self-assembled DNA nanostructures or self-assembled DNA origami, such as tetrahaeder structures and/or pillar structures.

In a method according to embodiments of the present invention, the exposing of the fiber optic sensor to the sample fluid may comprise contacting the fiber optic sensor to the sample fluid in a reaction chamber. For example, contacting the mass sensitive sensor to the sample fluid in a reaction chamber may comprise dipping the mass sensitive sensor, e.g. the fiber optic probe, in the sample fluid.

In a method according to embodiments of the present invention, exposing of the fiber optic sensor to the sample fluid may comprise performing a nucleic acid amplification of the plurality of different target molecules, such that the amplified target nucleic acids of the plurality of different target molecules form complexes with the corresponding plurality of distinct affinity probes.

In a method according to embodiments of the present invention, the nucleic acid amplification may comprise a Polymerase Chain Reaction, a Ligation Chain Reaction, a Rolling Circle Amplification, and/or an MNAzyme amplification.

Particular and preferred embodiments of the invention are set out in the appended dependent claims. Features from the dependent claims may be combined with features of the independent claim and with features of other dependent claims as appropriate within the scope of the claims.

These and other aspects of the invention will be apparent from and elucidated with reference to the embodiment(s) described hereinafter.

### Brief description of the drawings

FIG 1 illustrates an exemplary method in accordance with embodiments of the present invention.
FIG 2 schematically illustrates a method in accordance with embodiments of the present invention.
FIG 3 shows an exemplary sensor configuration for use in embodiments of the present invention.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or corresponding elements.

### Detailed description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of the invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

Where reference is made to a "sensor" is made in the present description, this refers to a sensing device, or sensing part thereof, to which target molecules of interest can bind such that the presence or absence of this bound molecule can be determined. Particularly, "sensor" may refer specifically to a mass-sensitive sensor, i.e. a device for measuring a property that functionally relates, e.g. bijectively relates, e.g. that scales proportionally, to mass associated with or bound to a sensitive surface thereof, e.g. a detection surface assembled with capture probes. The sensor may be adapted for submersion in a reaction chamber when the binding reaction occurs with such target molecules in a fluid sample to be analysed. The term "sensor" refers in present description specifically to a sensor used for analysis or detection of molecules whereby a target analyte interacts with an affinity probe and signals generated by this interaction are transformed by a physicochemical detection mechanism into a form that can be measured and quantified. An exemplary sensor may be an optical sensor, e.g. an optical biosensor, based on the principle of surface plasmon resonance (SPR), e.g. a fiber optic SPR (FO-SPR) device. Thus, the fiber optic sensor may comprise a fiber optic surface plasmon resonance device.

Where reference is made to "distinct" target molecules and "distinct" affinity probes hereinbelow, this refers to different such entities having a different chemical structure, e.g. substantially differing in chemical composition and/or chemical structure.

The present invention relates to a method for detecting a plurality of distinct target molecules in a sample fluid.

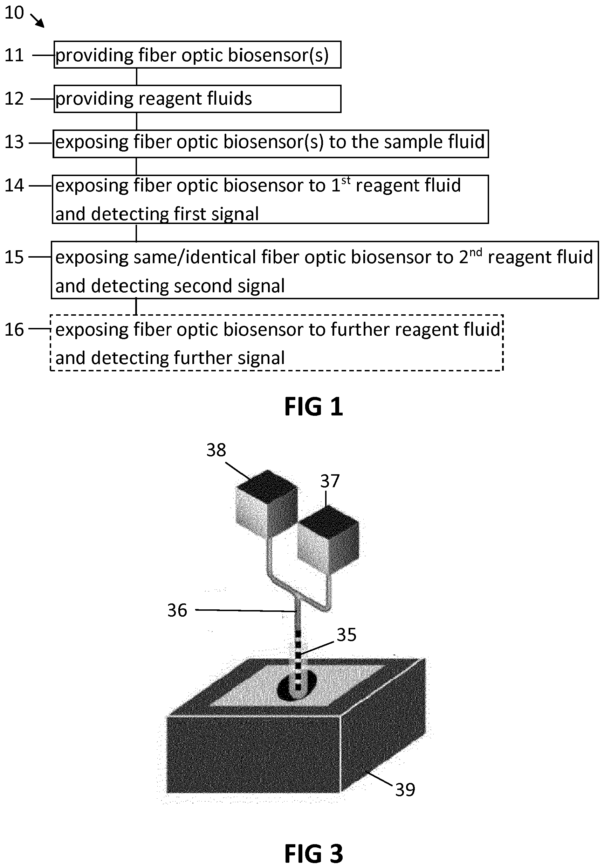

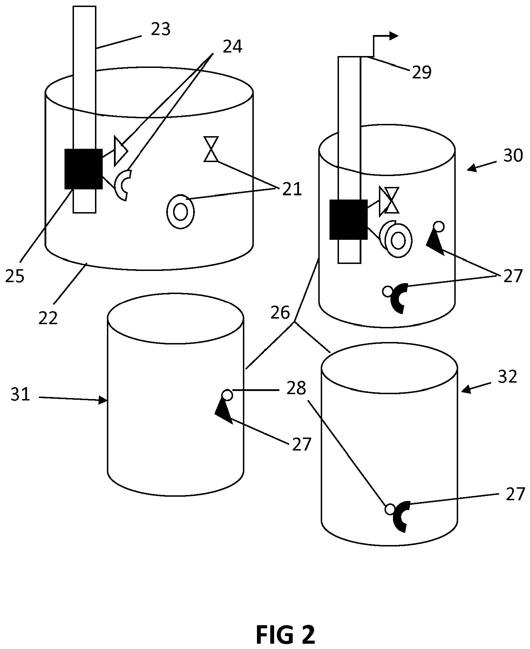

Referring to FIG 1 and FIG 2, an exemplary method 10 for detecting a plurality of distinct target molecules 21 in a sample fluid 22 according to embodiments of the present invention is illustrated. This method may be a method for multiplex target analysis to identify the target molecules of the plurality of distinct target molecules that are present in the sample fluid.

For example, the target molecules may comprise biomolecules, e.g. chemical entities produced by biological processes, and semi-synthetic or synthetic analogs thereof. Such biomolecules may comprise large macromolecules such as proteins (e.g. recombinant and/or natural proteins), carbohydrates, lipids, and/or nucleic acids. Such biomolecules may comprise small molecules such as primary metabolites, secondary metabolites, and natural products. Biomolecules may be endogenous or exogenous, e.g. the target molecules may comprise synthetic, semisynthetic and/or natural products, e.g. synthetic pharmaceuticals and/or biopharmaceuticals.

In a method according to embodiments of the present invention, the target molecules may comprise proteins to be detected in the sample fluid. It is an advantage of embodiments of the present invention that an efficient and specific detection of proteins is provided using fiber optic sensor probes, e.g. where disambiguation of binding of different target proteins on the sensor may be difficult using different melting curve characteristics.

The method 10 comprises the step of providing 11 at least one fiber optic sensor 23 functionalized with a plurality of distinct affinity probes 24 immobilized on a detection surface 25 of the fiber optic sensor 23. Such sensor 23 may combine affinity probes, e.g. comprising biologically-derived materials, with a physicochemical detector. Target analytes may interact with the affinity probes, and signals generated by this interaction may be measured using an optical sensor based on the principle of surface plasmon resonance (SPR), e.g. a fiber optic SPR (FO-SPR) device. Thus, the fiber optic sensor may comprise a fiber optic surface plasmon resonance device. The sensor may be a mass-based sensor.

For example, target molecules may be identified by DNA melting peak analysis using such mass based sensor, as known in the art. For example, providing the at least one fiber optic sensor 11 may comprise providing a fiber optic sensor configured for detecting an optical signal sensitive to surface plasmon resonance on the detection surface due to simultaneous binding of the target molecules 21 with the corresponding distinct affinity probes 24 and the corresponding affinity reagents discussed further hereinbelow, e.g. antibodies may be bound to DNA and this DNA may hybridize on the detection surface. A signal 29, e.g. the first signal, the second signal and/or any further signal referred to hereinbelow, may be generated by increasing a temperature of the fiber optic sensor, e.g. a fiber optic surface plasmon resonance sensor, and determining a change in the optical signal representative of a temperature-induced release of the target molecules 21 from the detection surface 25, e.g. a melting of the hybridized DNA. This increasing of the temperature of the fiber optic sensor may furthermore be used advantageously to regenerate the detection surface of the fiber optic sensor, e.g. such that the same fiber optic sensor may be used in a further detection step.

In a method according to embodiments of the present invention, the detection surface 25 of the at least one fiber optic sensor may be provided with a surface texturing structure to enable binding of the target molecules on the corresponding affinity probes in a predetermined orientation. Such surface texturing structure may comprise self-assembled DNA structures, e.g. self-assembled DNA nanostructures or self-assembled DNA origami, such as tetrahaeder structures and/or pillar structures.

Each affinity probe 24 specifically binds to a corresponding target molecule 21 of the plurality of distinct target molecules to be detected in the sample fluid 22. In embodiments where the at least one fiber optic sensor 23 comprises a plurality of fiber optic sensors, each of the plurality of fiber optic sensors 23 is functionalized with a same plurality of distinct affinity probes, e.g. comprises the same set of distinct affinity probes for specifically binding to the same set of corresponding target molecules. It is an advantage of embodiments of the present invention that identically manufactured fiber optic sensors 23 can be used to test for a possibly large plurality of target molecules in a target-specific manner.

In the method according to the present invention, providing the at least one fiber optic sensor comprises providing the at least one fiber optic sensor functionalized with a plurality of distinct affinity probes comprising an antibody, a monoclonal antibody, an aptamer, an affimer, a peptide, a nucleic acid and/or another small molecule that specifically binds to a target molecule of the plurality of target molecules.

It is an advantage of embodiments of the present invention that a high efficiency can be achieved due to the use of multi-purpose, e.g. identically manufactured, sensors, and/or by using a single fluid sample to test for a possibly large plurality of different target molecules. It is, for example, an advantage of a single fluid sample to test for a plurality of different target molecules that measurement errors and/or systematic errors induced by the preparation of different samples of the same material can be avoided. It is a further advantage that, in accordance with embodiments of the present invention, a reference measurement and/or positive control can be performed within the same measurement.

For example, FIG 3 illustrates an exemplary sensor for use in embodiments of the present invention. This sensor comprises a fiber optic surface plasmon resonance (FO-SPR) tip 35 for exposing to the sample fluid, e.g. by means of dipping in a reservoir. This tip 35 may be connected to a bifurcated optical fiber 36, one bifurcation of this fiber being connected to a light source 37, and the other bifurcation of this fiber being connected to a detector for generating a detection signal 29, e.g. a spectrometer 38. The tip 35 may be directly or indirectly, e.g. via a fluid medium, in thermal contact with a controlled heat source, e.g. a thermocycler 39, e.g. such as to obtain a detection and/or quantitative measurement by melting curve analysis.

The method further comprises providing 12 a plurality of reagent fluids 26. Each reagent fluid 26 comprises at least one affinity reagent 27 for binding, simultaneously with a corresponding affinity probe 24 of the plurality of distinct affinity probes, to the corresponding target molecule 21. Thus, the affinity reagent 27 may bind to a corresponding target molecule 21, while this target molecule 21 can bind in parallel to a corresponding affinity probe 24 immobilized on the detection surface 25 of the sensor 23.

Each affinity reagent 27 is bound to a metal nanoparticle 28 for influencing a signal 29 generated by the fiber optic sensor 23 when the affinity reagent 27 and the corresponding affinity probe 24 on the detection surface 25 of the fiber optic sensor 23 are simultaneously bound to the corresponding target molecule 21.

Such nanoparticle 28 may be a mass element adapted for increasing the sensitivity of a binding assay. For example, the nanoparticle 28 may enhance the signal 29 which may be detected by the sensor. For example, the nanoparticle may comprise a gold nanoparticle (Au NP), which may be advantageous for enhancing a signal generated by a fiber optic surface plasmon resonance sensor. Other exemplary nanoparticle materials, which may also be advantageously used in combination with a fiber optic SPR sensor, comprise silver, copper, platinum, ruthenium, palladium, or an alloy or combination of such metals. The nanoparticle may have a diameter in the range of 2 nm to 100 nm.

In a method according to the present invention, the at least one affinity reagent comprises an antibody, a monoclonal antibody, an aptamer, an affimer, a peptide, a nucleic acid and/or another small molecule that specifically binds to a target molecule of the plurality of target molecules in parallel with the binding of a corresponding affinity probe of the plurality of distinct affinity probes to the target molecule.

Each reagent fluid 26 has a different composition than each other reagent fluid such as to enable binding of the affinity reagent 27 or affinity reagents in each reagent fluid 26 to a different target molecule 21 or to a different combination of target molecules.

The method 10 also comprises exposing 13 the or each fiber optic sensor 23 to the sample fluid 22 to bind the different target molecules 21, when present in the sample fluid 22, to the corresponding plurality of distinct affinity probes 24 immobilized on the detection surface 25 of the fiber optic sensor 23. Exposing 13 the fiber optic sensor to the sample fluid may comprise contacting the fiber optic sensor to the sample fluid in a reaction chamber. For example, contacting the sensor to the sample fluid in a reaction chamber may comprise dipping the fiber optic sensor, e.g. a mass sensitive sensor such as a fiber optic surface plasmon resonance sensor, in the sample fluid.

In a method according to embodiments of the present invention, exposing of the fiber optic sensor to the sample fluid may comprise performing a nucleic acid amplification of the plurality of different target molecules, such that the amplified target nucleic acids of the plurality of different target molecules form complexes with the corresponding plurality of distinct affinity probes. For example, such nucleic acid amplification may comprise a Polymerase Chain Reaction, a Ligation Chain Reaction, a Rolling Circle Amplification, and/or an MNAzyme amplification.

The method 10 further comprises exposing 14 a first fiber optic sensor of the at least one fiber optic sensor 23 to a first reagent fluid 30 of the plurality of reagent fluids 26, and detecting a first signal 29 generated by the first fiber optic sensor 27 to determine whether a first target molecule or any of a first combination of target molecules was present in the sample fluid 26. This step of exposing 14 the first sensor to the first reagent fluid may be performed after the step of exposing 13 at least the first sensor to the sample fluid.

In the method according to the present invention, the first reagent fluid comprises, for each target molecule of the plurality of target molecules, a corresponding affinity reagent for binding simultaneously with a corresponding affinity probe of the plurality of distinct affinity probes to the target molecule, such that the first detected signal forms a signal indicative of the presence of any of the plurality of target molecules in the sample liquid. It is an advantage of embodiments of the present invention that a quick screening can be obtained for a large number of target molecules, while allowing an efficient identification of the present target molecules in a stepwise approach.

The method also comprises exposing 15 the first fiber optic sensor, or a second fiber optic sensor of the at least one fiber optic sensor 23, e.g. the same or an identical fiber optic sensor as was exposed 14 to the first reagent fluid 30, to a second reagent fluid 31 of the plurality of reagent fluids 26, and detecting a second signal 29 generated by that optic sensor, e.g. the first fiber optic sensor or respectively of the second fiber optic sensor, to determine whether a second target molecule or any of a second combination of target molecules was present in the sample fluid.

The steps of exposing 14 to the first reagent fluid and exposing 15 to the second reagent fluid may be performed consecutively. For example, the steps of exposing the sensor or sensors to the sample fluid, exposing 14 the first sensor to the first reagent fluid and exposing 15 the first or second sensor to the second reagent fluid may be performed consecutively. Particularly, the step of exposing 15 the first or second sensor to the second reagent fluid may take the first signal generated by exposing the first sensor to the first reagent fluid into account, for example to determine whether the same or another identical sensor is exposed to the second reagent fluid, and/or to select the second reagent fluid from the plurality of reagent fluids taking the first signal into account.

In the method according to the present invention, if the first signal indicated the presence of any of the first combination of target molecules, the specific target molecule or molecules of the first combination that are present in the sample fluid may be further determined by the second signal, wherein the affinity reagent or affinity reagents in the second reagent fluid form a first proper subset of the affinity reagents in the first reagent fluid. In present disclosure, a proper subset refers to the mathematical concept in which every component affinity reagent present in the second reagent fluid was also present in the first reagent fluid, but there exists at least one component affinity reagent in the first reagent fluid that is not present in the second reagent fluid. It is an advantage of embodiments of the present invention that molecules not present in the sample fluid can be eliminated quickly by stepwise testing.

According to the present invention, the first, or respectively the second, fiber optic sensor may be exposed to the second reagent fluid if, e.g. if and only if, the first signal indicated a presence of the first target molecule or of any of the target molecules of the first combination of target molecules, e.g. to determine the target molecules present in the sample more accurately.

In a method according to embodiments of the present invention, the at least one fiber optic sensor may comprise a plurality of fiber optic sensors, wherein the second fiber optic sensor is exposed to the second reagent fluid if the first signal indicated a presence of the first target molecule or of any target molecule of the first combination of target molecules.

In another method according to embodiments of the present invention, the first fiber optic sensor may be exposed to the second reagent fluid if, e.g. if and only if, the first signal indicated an absence of the first target molecule or of all target molecules of the first combination of target molecules. For example, it is an advantage of embodiments of the present invention that a fiber optic sensor can be easily reused for further testing if no binding was to molecules to be tested was observed. Furthermore, this may apply equally to an exposure to a further reagent fluid as described further hereinbelow.

For example, the first fiber optic sensor may be exposed to the second reagent fluid if, e.g. if and only if, the first signal indicated a presence of the first target molecule or of any of the target molecules of the first combination of target molecules, e.g. to determine the target molecules present in the sample more accurately. Assuming a presence any first target molecule, the second reagent fluid may contain a proper subset of the affinity reagents present in the first reagent fluid. Assuming a presence of any second target molecule, a further reagent fluid may contain a proper subset of the affinity reagents present in the second reagent fluid. However, in absence of all second target molecules, a further reagent fluid may contain a complimentary proper subset of the affinity reagents present in the first reagent fluid, e.g. complimentary to the first proper subset formed by the affinity reagents present in the second reagent fluid. This principle of stepwise elimination and/or more specific testing may be applied in yet further steps to test yet further reagent fluids.

In a method according to embodiments of the present invention, the first reagent fluid may comprise, for each target molecule of the plurality of target molecules, a corresponding affinity reagent for binding simultaneously with a corresponding affinity probe of the plurality of distinct affinity probes to the target molecule, such that the first detected signal forms a global screening signal indicative of the presence of any of the plurality of target molecules in the sample liquid.

A method according to embodiments of the present invention may further comprise exposing 16 the, or any of the, at least one fiber optic sensor to a further reagent fluid 32 of the plurality of reagent fluids, and detecting a further signal 29 generated by the thus exposed fiber optic sensor to determine whether at least one further target molecule or any of a further combination of target molecules was present in the sample fluid. This further step may be performed after the step of exposing 15 the sensor to the second reagent fluid. Again, in a method according to embodiments of the present invention, if the second signal indicated the presence of any of the second combination of target molecules, the specific target molecule or molecules of the second combination that are present in the sample fluid may be further determined by the further signal, in which the affinity reagent or affinity reagents in the further reagent fluid form a second proper subset of the affinity reagents in the second reagent fluid.

Thus, in a method according to embodiments of the present invention, the affinity reagent or affinity reagents in the further reagent fluid may form a second proper subset of the affinity reagents in the first reagent fluid, in which this second proper subset is complimentary to the first proper subset.

In a method according to embodiments of the present invention, the number of affinity reagents in the second proper subset may be in the range of 50% to 150% of the number of affinity reagents in the first proper subset. For example, if the absence of all affinity reagents in the second combination of target molecules was detected, the second proper subset formed by the affinity reagents in the further reagent fluid may be selected as a complimentary proper subset of the affinity reagents in the first reagent fluid. In such case, each of the first proper subset and the second proper subset may consist of complimentary subsets of the affinity reagents present in the first reagent fluid, in which the first proper subset and the second proper subset contain an about equal number of affinity reagents.

Thus, it is an advantage of embodiments of the present invention that molecules not present in the sample fluid can be eliminated quickly by stepwise testing for complimentary sets of about equal size.

It is an advantage of embodiments of the present invention that identical fiber optic sensors can be used, while allowing a specific testing for a plurality of molecules of interest in a stepwise procedure. It is a further advantage that distinct fiber optic sensors need not to be manufactured and functionalized specifically to test for different molecules.

In a method in accordance with embodiments of the present invention, the fiber optic sensor, e.g. comprising an optic fiber tip having a detection surface on which the plurality of distinct affinity probes are immobilized, may be exposed to the sample fluid to bind the different target molecules, e.g. to bind the target molecules in the sample fluid to the distinct affinity probes by exposing the optic fiber tip to the sample fluid.

In examples not falling under the scope of the present invention, the method may comprise, after exposing the fiber optic sensor to the sample fluid, e.g. dipping the fiber optic tip in the sample fluid, a step of moving the fiber optic sensor, e.g. the optic fiber tip, to the first reagent fluid and exposing the fiber optic sensor, e.g. the optic fiber tip, to the first reagent fluid. This moving and exposing may comprise a sequential exposure of the fiber optic sensor to a plurality of different reagent fluids.

For example, this moving and exposing may be performed using a three-dimensional positioning system, e.g. an actuated frame having at least three degrees of freedom, e.g. three degrees of translation. Such positioning system may comprise a controller, controlling the motion of the fiber optic sensor from the sample fluid to the reagent fluid or along a sequence of reagent fluids. However, simultaneously, another fiber optic sensor may be moved between the sample (or another sample) and the reagent fluids, and exposed thereto, in an synchronized manner, e.g. such as to avoid collision of the former fiber optic sensor and the other fiber optic sensor. Therefore, a three-dimensional positioning system, e.g. a positioning robot, may be controlled to move one or more of fiber optic sensors. Thus, an efficient and flexible use of a plurality of sample fluids, fiber optic sensors and reagent fluids can be achieved, e.g. by runtime optimized control of the motion sequence of one or more fiber optic sensors.

## Claims

1. A method (10) for detecting a plurality of distinct target molecules (21) in a sample fluid (26), the method comprising:
- providing (11) at least one fiber optic sensor (23) having a detection surface (25), the sensor being configured for detecting an optical signal (29) sensitive to surface plasmon resonance on the detection surface (25), and functionalized with a plurality of distinct affinity probes (24) selected from an antibody, a monoclonal antibody, an aptamer, an affimer, a peptide, a nucleic acid and/or another small molecule that specifically binds to a target molecule of the plurality of distinct target molecules (21), said affinity probes (24) being immobilized on the detection surface (25) of said fiber optic sensor (23), each affinity probe (24) specifically binding to a corresponding target molecule of said plurality of distinct target molecules (21) to be detected in said sample fluid (26), wherein, if the at least one fiber optic sensor (23) comprises a plurality of fiber optic sensors, each of said plurality of fiber optic sensors is functionalized with a same plurality of distinct affinity probes (24);
- providing (12) a plurality of reagent fluids (26), each reagent fluid comprising a plurality of affinity reagents (27) selected from an antibody, a monoclonal antibody, an aptamer, an affimer, a peptide, a nucleic acid and/or another small molecule that specifically binds to a target molecule of the plurality of distinct target molecules (21) in parallel with said binding of a corresponding affinity probe of the plurality of distinct affinity probes (24) to said target molecule (21, each affinity reagent being bound to a metal nanoparticle (28) for enhancing the optical signal (29) due to simultaneous binding of a target molecule (21) with a corresponding distinct affinity probe (24) and at least one of the corresponding affinity reagents (27), wherein each reagent fluid (26) has a different composition than each other reagent fluid such as to enable binding of the affinity reagent or affinity reagents (27) in each reagent fluid (26) to a different target molecule (21) or to a different combination of target molecules;
- exposing (13) the or each fiber optic sensor (23) to said sample fluid (22) to bind said different target molecules (21), when present in the sample fluid, to said corresponding plurality of distinct affinity probes (24);
- exposing (14) a first fiber optic sensor of the at least one fiber optic sensors (23) to a first reagent fluid (30) of said plurality of reagent fluids (26), and detecting a first optical signal generated by said first fiber optic sensor (23) to determine whether a first target molecule or any of a first combination of target molecules is present in the sample fluid (22); and
- exposing (15) the same first fiber optic sensor, or an identical second fiber optic sensor of the at least one fiber optic sensors (23) to a second reagent fluid (31) of said plurality of reagent fluids (26), and detecting a second optical signal generated by the first fiber optic sensor, or respectively by the second fiber optic sensor, to determine whether a second target molecule or any of a second combination of target molecules is present in the sample fluid (22),
wherein the reagent fluids (30,31) are in thermal contact with a controlled heat source (39) and said optical signals (29) are monitored while increasing a temperature of said detection surface (25) and wherein a change in the monitored optical signal (29) that is representative of a temperature-induced release of the target molecules (21) from the detection surface (25) is detected to determine whether the target molecules are present in the sample fluid (22) and are bound to the detection surface by affinity probes and affinity reagents,
wherein, if said first signal indicates the presence of any of said first combination of target molecules, the specific target molecule or molecules of said first combination present in the sample fluid (22) is further determined by said second signal, wherein the affinity reagents in the second reagent fluid (31) form a first proper subset of the affinity reagents in the first reagent fluid (30).

2. The method according to claim 1, further comprising exposing the or any of the at least one fiber optic sensors (23) to a further reagent fluid (32) of said plurality of reagent fluids, and detecting a further optical signal generated by the thus exposed fiber optic sensor to determine whether at least one further target molecule or any of a further combination of target molecules is present in the sample fluid (22).

3. The method according to claim 2, wherein the affinity reagents in the further reagent fluid (32) form a second proper subset of the affinity reagents in the first reagent fluid (30), said second proper subset being complimentary to said first proper subset.

4. The method according to claim 3, wherein the number of affinity reagents in said second proper subset is in the range of 50% to 150% of the number of affinity reagents in the first proper subset.

5. The method according to any of the previous claims, wherein the first reagent fluid (30) comprises, for each target molecule of the plurality of target molecules (21), a corresponding affinity reagent for binding simultaneously with a corresponding affinity probe of the plurality of distinct affinity probes (24) to said target molecule, such that the first detected signal forms a signal indicative of the presence of any of the plurality of target molecules (21) in the sample liquid (22).

6. The method according to any of the previous claims, wherein said target molecules (21) comprise proteins to be detected in said sample fluid (22).

7. The method according to claim 1, wherein said detection surface (25) of the at least one fiber optic sensor (23) is provided with a surface texturing structure to enable binding of the target molecules (21) on the corresponding affinity probes (24) in a predetermined orientation.

8. The method according to claim 7, wherein said surface texturing structure comprises self-assembled DNA structures.

9. The method according to any of the previous claims, in which said exposing (13) the fiber optic sensor (23) to said sample fluid (22) comprises contacting the fiber optic sensor (23) to the sample fluid (22) in a reaction chamber.

10. The method according to any of the previous claims, wherein said exposing of (13) the fiber optic sensor (23) to said sample fluid (22) comprises performing a nucleic acid amplification of the plurality of different target molecules, such that the amplified target nucleic acids of the plurality of different target molecules (21) form complexes with said corresponding plurality of distinct affinity probes (24).

11. The method according to claim 10, wherein said nucleic acid amplification comprises a Polymerase Chain Reaction, a Ligation Chain Reaction, a Rolling Circle Amplification, and/or an MNAzyme amplification.

## Patentansprüche

1. Verfahren (10) zum Nachweis einer Vielzahl unterschiedlicher Zielmoleküle (21) in einer Probenflüssigkeit (26), wobei das Verfahren umfasst:
- Bereitstellen (11) mindestens eines faseroptischen Sensors (23) mit einer Detektionsoberfläche (25), wobei der Sensor zum Detektieren eines optischen Signals (29) konfiguriert ist, das für Oberflächenplasmonenresonanz auf der Detektionsoberfläche (25) empfindlich ist, und funktionalisiert ist mit a Vielzahl unterschiedlicher Affinitätssonden (24), ausgewählt aus einem Antikörper, einem monoklonalen Antikörper, einem Aptamer, einem Affimer, einem Peptid, einer Nukleinsäure und/oder einem anderen kleinen Molekül, das spezifisch an ein Zielmolekül der Vielzahl unterschiedlicher Zielmoleküle bindet ( 21), wobei die Affinitätssonden (24) auf der Detektionsoberfläche (25) des faseroptischen Sensors (23) immobilisiert sind, wobei jede Affinitätssonde (24) spezifisch an ein entsprechendes Zielmolekül der Vielzahl von unterschiedlichen Zielmolekülen (21) bindet, in dem Probenfluid (26) zu detektieren, wobei, wenn der mindestens eine faseroptische Sensor (23) eine Vielzahl von faseroptischen Sensoren umfasst, jeder der Vielzahl von faseroptischen Sensoren mit einer gleichen Vielzahl von unterschiedlichen Affinitätssonden funktionalisiert ist (24);
- Bereitstellen (12) einer Vielzahl von Reagenzflüssigkeiten (26), wobei jede Reagenzflüssigkeit eine Vielzahl von Affinitätsreagenzien (27) umfasst, ausgewählt aus einem Antikörper, einem monoklonalen Antikörper, einem Aptamer, einem Affimer, einem Peptid, einer Nukleinsäure und/oder ein weiteres kleines Molekül, das spezifisch an ein Zielmolekül der mehreren unterschiedlichen Zielmoleküle (21) parallel zu der Bindung einer entsprechenden Affinitätssonde der mehreren unterschiedlichen Affinitätssonden (24) an das Zielmolekül (21, jedes Affinitätsreagenz) bindet an ein Metallnanopartikel (28) gebunden ist, um das optische Signal (29) aufgrund gleichzeitiger Bindung eines Zielmoleküls (21) mit einer entsprechenden unterschiedlichen Affinitätssonde (24) und mindestens einem der entsprechenden Affinitätsreagenzien (27) zu verstärken, wobei jedes Reagenzfluid (26) eine andere Zusammensetzung als jedes andere Reagenzfluid hat, um eine Bindung des Affinitätsreagenz oder der Affinitätsreagenzien (27) in jedem Reagenzfluid (26) an ein anderes Zielmolekül (21) oder an ein anderes zu ermöglichen Kombination von Zielmolekülen;
- Aussetzen (13) des oder jedes faseroptischen Sensors (23) an die Probenflüssigkeit (22), um die unterschiedlichen Zielmoleküle (21), wenn sie in der Probenflüssigkeit vorhanden sind, an die entsprechende Vielzahl von unterschiedlichen Affinitätssonden (24) zu binden;
- Aussetzen (14) eines ersten faseroptischen Sensors des mindestens einen faseroptischen Sensors (23) einem ersten Reagensfluid (30) der mehreren Reagensfluide (26) und Erfassen eines ersten optischen Signals, das von der ersten Faser erzeugt wird optischer Sensor (23) zum Bestimmen, ob ein erstes Zielmolekül oder irgendeines einer ersten Kombination von Zielmolekülen in der Probenflüssigkeit (22) vorhanden ist; Und
- Aussetzen (15) desselben ersten faseroptischen Sensors oder eines identischen zweiten faseroptischen Sensors des mindestens einen faseroptischen Sensors (23) einem zweiten Reagenzfluid (31) der Vielzahl von Reagenzfluiden (26), und Detektieren eines zweiten optischen Signals, das von dem ersten faseroptischen Sensor bzw. von dem zweiten faseroptischen Sensor erzeugt wird, um zu bestimmen, ob ein zweites Zielmolekül oder eines einer zweiten Kombination von Zielmolekülen in der Probenflüssigkeit (22) vorhanden ist,
wobei die Reagensflüssigkeiten (30, 31) in thermischem Kontakt mit einer gesteuerten Wärmequelle (39) sind und die optischen Signale (29) überwacht werden, während eine Temperatur der Erfassungsoberfläche (25) erhöht wird, und wobei eine Änderung des überwachten optischen Signals erfolgt (29), das repräsentativ für eine temperaturinduzierte Freisetzung der Zielmoleküle (21) von der Detektionsoberfläche (25) ist, erfasst wird, um zu bestimmen, ob die Zielmoleküle in der Probenflüssigkeit (22) vorhanden und an die Detektionsoberfläche gebunden sind durch Affinitätssonden und Affinitätsreagenzien,
wobei, wenn das erste Signal das Vorhandensein irgendeines der ersten Kombination von Zielmolekülen anzeigt, das spezifische Zielmolekül oder die spezifischen Zielmoleküle der ersten Kombination, die in der Probenflüssigkeit (22) vorhanden sind, ferner durch das zweite Signal bestimmt wird, wobei die Affinitätsreagenzien in das zweite Reagenzfluid (31) eine erste geeignete Teilmenge der Affinitätsreagenzien in dem ersten Reagenzfluid (30) bildet.

2. Verfahren nach Anspruch 1, ferner umfassend das Aussetzen des oder irgendeines der mindestens einen faseroptischen Sensoren (23) einem weiteren Reagenzfluid (32) der Vielzahl von Reagenzfluiden und Detektieren eines weiteren optischen Signals, das dadurch erzeugt wird den so freigelegten faseroptischen Sensor, um zu bestimmen, ob mindestens ein weiteres Zielmolekül oder irgendeine weitere Kombination von Zielmolekülen in der Probenflüssigkeit (22) vorhanden ist.

3. Verfahren nach Anspruch 2, wobei die Affinitätsreagenzien in der weiteren Reagenzflüssigkeit (32) eine zweite richtige Teilmenge der Affinitätsreagenzien in der ersten Reagenzflüssigkeit (30) bilden, wobei die zweite richtige Teilmenge komplementär zu der ersten richtigen Teilmenge ist.

4. Verfahren nach Anspruch 3, wobei die Anzahl der Affinitätsreagenzien in der zweiten richtigen Untergruppe im Bereich von 50% bis 150% der Anzahl der Affinitätsreagenzien in der ersten richtigen Untergruppe liegt.

5. Verfahren nach einem der vorhergehenden Ansprüche, wobei das erste Reagenzfluid (30) für jedes Zielmolekül der Vielzahl von Zielmolekülen (21) ein entsprechendes Affinitätsreagenz zum gleichzeitigen Binden mit einer entsprechenden Affinitätssonde umfasst eine Vielzahl unterschiedlicher Affinitätssonden (24) zu dem Zielmolekül, so dass das erste detektierte Signal ein Screening-Signal bildet, das die Anwesenheit irgendeines der Vielzahl von Zielmolekülen (21) in der Probenflüssigkeit (22) anzeigt.

6. Verfahren nach einem der vorhergehenden Ansprüche, wobei die Zielmoleküle (21) in der Probenflüssigkeit (22) nachzuweisende Proteine umfassen.

7. Verfahren nach Anspruch 1, wobei die Detektionsoberfläche (25) des mindestens einen faseroptischen Sensors (23) mit einer Oberflächentexturierungsstruktur versehen ist, um eine Bindung der Zielmoleküle (21) an die entsprechenden Affinitätssonden (21) zu ermöglichen. 24) in einer vorbestimmten Ausrichtung.

8. Verfahren nach Anspruch 7, wobei die Oberflächentexturierungsstruktur selbstorganisierte DNA-Strukturen umfasst.

9. Verfahren nach einem der vorhergehenden Ansprüche, bei dem das Aussetzen (13) des faseroptischen Sensors (23) dem Probenfluid (22) das Kontaktieren des faseroptischen Sensors (23) mit dem Probenfluid (22) umfasst eine Reaktionskammer.

10. Verfahren nach einem der vorhergehenden Ansprüche, wobei das Aussetzen ( 13 ) des faseroptischen Sensors ( 23 ) dem Probenfluid ( 22 ) das Durchführen einer Nukleinsäureamplifikation der Vielzahl unterschiedlicher Zielmoleküle umfasst, so dass die amplifizierte Zielnucleinsäuren der Vielzahl unterschiedlicher Zielmoleküle (21) bilden Komplexe mit der entsprechenden Vielzahl unterschiedlicher Affinitätssonden (24).

11. Verfahren nach Anspruch 10, wobei die Nukleinsäure-Amplifikation eine Polymerase-Kettenreaktion, eine Ligations-Kettenreaktion, eine Rolling-Circle-Amplifikation und/oder eine MNAzyme-Amplifikation umfasst.

## Revendications

1. Procédé (10) pour détecter une pluralité de molécules cibles distinctes (21) dans un fluide échantillon (26), le procédé comprenant :
- prévoir (11) au moins un capteur à fibre optique (23) ayant une surface de détection (25), le capteur étant configuré pour détecter un signal optique (29) sensible à la résonance plasmon de surface sur la surface de détection (25) et fonctionnalisé avec un pluralité de sondes d'affinité distinctes (24) choisies parmi un anticorps, un anticorps monoclonal, un aptamère, un affimère, un peptide, un acide nucléique et/ou une autre petite molécule qui se lie spécifiquement à une molécule cible de la pluralité de molécules cibles distinctes ( 21), lesdites sondes d'affinité (24) étant immobilisées sur la surface de détection (25) dudit capteur à fibre optique (23), chaque sonde d'affinité (24) se liant spécifiquement à une molécule cible correspondante de ladite pluralité de molécules cibles distinctes (21) à détecter dans ledit fluide échantillon (26), dans lequel, si le au moins un capteur à fibre optique (23) comprend une pluralité de capteurs à fibre optique, chacun de ladite pluralité de capteurs à fibre optique est fonctionnalisé avec une même pluralité de sondes d'affinité distinctes (24);
- fournir (12) une pluralité de fluides réactifs (26), chaque fluide réactif comprenant une pluralité de réactifs d'affinité (27) choisis parmi un anticorps, un anticorps monoclonal, un aptamère, un affimère, un peptide, un acide nucléique et/ou une autre petite molécule qui se lie spécifiquement à une molécule cible de la pluralité de molécules cibles distinctes (21) parallèlement à ladite liaison d'une sonde d'affinité correspondante de la pluralité de sondes d'affinité distinctes (24) à ladite molécule cible (21, chaque réactif d'affinité étant lié à une nanoparticule métallique (28) pour améliorer le signal optique (29) en raison de la liaison simultanée d'une molécule cible (21) avec une sonde d'affinité distincte correspondante (24) et au moins un des réactifs d'affinité correspondants (27), dans lequel chaque fluide réactif (26) a une composition différente de chaque autre fluide réactif de manière à permettre la liaison du réactif d'affinité ou des réactifs d'affinité (27) dans chaque fluide réactif (26) à une molécule cible différente (21) ou à une molécule cible différente combinaison de molécules cibles;
- exposer (13) le ou chaque capteur à fibre optique (23) audit fluide échantillon (22) pour lier lesdites différentes molécules cibles (21), lorsqu'elles sont présentes dans le fluide échantillon, à ladite pluralité correspondante de sondes d'affinité distinctes (24) ;
- exposer (14) un premier capteur à fibre optique du ou des capteurs à fibre optique (23) à un premier fluide réactif (30) de ladite pluralité de fluides réactifs (26), et détecter un premier signal optique généré par ladite première fibre un capteur optique (23) pour déterminer si une première molécule cible ou l'une quelconque d'une première combinaison de molécules cibles est présente dans le fluide échantillon (22) ; et
- exposer (15) le même premier capteur à fibre optique, ou un second capteur à fibre optique identique du au moins un capteur à fibre optique (23), à un second fluide réactif (31) de ladite pluralité de fluides réactifs (26), et détecter un second signal optique généré par le premier capteur à fibre optique, ou respectivement par le second capteur à fibre optique, pour déterminer si une seconde molécule cible ou l'une quelconque d'une seconde combinaison de molécules cibles est présente dans le fluide échantillon (22),
dans lequel les fluides réactifs (30, 31) sont en contact thermique avec une source de chaleur contrôlée (39) et lesdits signaux optiques (29) sont contrôlés tout en augmentant une température de ladite surface de détection (25) et dans lequel un changement du signal optique contrôlé (29) qui est représentatif d'une libération induite par la température des molécules cibles (21) à partir de la surface de détection (25) est détectée pour déterminer si les molécules cibles sont présentes dans le fluide échantillon (22) et sont liées à la surface de détection par des sondes d'affinité et des réactifs d'affinité,
dans lequel, si ledit premier signal indique la présence de l'une quelconque de ladite première combinaison de molécules cibles, la ou les molécules cibles spécifiques de ladite première combinaison présentes dans l'échantillon de fluide (22) sont en outre déterminées par ledit second signal, dans lequel les réactifs d'affinité dans le second fluide réactif (31) forme un premier sous-ensemble approprié des réactifs d'affinité dans le premier fluide réactif (30).

2. Procédé selon la revendication 1, comprenant en outre l'exposition du ou de l'un quelconque des au moins un capteurs à fibre optique (23) à un autre fluide réactif (32) de ladite pluralité de fluides réactifs, et la détection d'un autre signal optique généré par le capteur à fibre optique ainsi exposé, pour déterminer si au moins une autre molécule cible ou l'une quelconque d'une autre combinaison de molécules cibles est présente dans le fluide échantillon (22).

3. Procédé selon la revendication 2, dans lequel les réactifs d'affinité dans l'autre fluide réactif (32) forment un deuxième sous-ensemble approprié des réactifs d'affinité dans le premier fluide réactif (30), ledit deuxième sous-ensemble approprié étant complémentaire audit premier sous-ensemble approprié.

4. Procédé selon la revendication 3, dans lequel le nombre de réactifs d'affinité dans ledit deuxième sous-ensemble approprié est dans la plage de 50 % à 150 % du nombre de réactifs d'affinité dans le premier sous-ensemble approprié.

5. Procédé selon l'une quelconque des revendications précédentes, dans lequel le premier fluide réactif (30) comprend, pour chaque molécule cible de la pluralité de molécules cibles (21), un réactif d'affinité correspondant pour se lier simultanément avec une sonde d'affinité correspondante du pluralité de sondes d'affinité distinctes (24) à ladite molécule cible, de sorte que le premier signal détecté forme un signal de criblage indicatif de la présence de l'une quelconque de la pluralité de molécules cibles (21) dans l'échantillon liquide (22).

6. Procédé selon l'une quelconque des revendications précédentes, dans lequel lesdites molécules cibles (21) comprennent des protéines à détecter dans ledit fluide échantillon (22).

7. Procédé selon la revendication 1, dans lequel ladite surface de détection (25) du au moins un capteur à fibre optique (23) est pourvue d'une structure de texturation de surface pour permettre la fixation des molécules cibles (21) sur les sondes d'affinité correspondantes ( 24) dans une orientation prédéterminée.

8. Procédé selon la revendication 7, dans lequel ladite structure de texturation de surface comprend des structures d'ADN auto-assemblées.

9. Procédé selon l'une quelconque des revendications précédentes, dans lequel ladite exposition (13) du capteur à fibre optique (23) audit fluide échantillon (22) comprend la mise en contact du capteur à fibre optique (23) avec le fluide échantillon (22) dans une chambre de réaction.

10. Procédé selon l'une quelconque des revendications précédentes, dans lequel ladite exposition (13) du capteur à fibre optique (23) audit fluide échantillon (22) comprend la réalisation d'une amplification d'acide nucléique de la pluralité de molécules cibles différentes, de sorte que la les acides nucléiques cibles amplifiés de la pluralité de molécules cibles différentes (21) forment des complexes avec ladite pluralité correspondante de sondes d'affinité distinctes (24).

11. Procédé selon la revendication 10, dans lequel ladite amplification d'acide nucléique comprend une réaction en chaîne par polymérase, une réaction en chaîne par ligature, une amplification en cercle roulant et/ou une amplification MNAzyme.
